# EUROPEAN PATENT APPLICATION

(11) **EP 1 676 836 A1**
(43) Date of publication of application: **05.07.2006**
(21) Application number: 04380295.8
(22) Date of filing: 30.12.2004
(51) Int. Cl.: C07D 205/12

(54) **Regioselective hydroxylation, functionalisation and protection of spirolactams II**

(71) Applicant: LABORATORIOS DEL DR. ESTEVE, S.A., 08041 Barcelona (ES)
(72) Inventor: Noheda Marín, Pedro, Juan de la Cierva 3 28006 Madrid (ES); Benito Arenas, Raúl, Juan de la Cierva 3 28006 Madrid (ES); Maroto Quintana, Sergio, Juan de la Cierva 3 28006 Madrid (ES); Bernabé Pajares, Manuel, Juan de la Cierva 3 28006 Madrid (ES); Tabares Cantero, Nuria, Juan de la Cierva 3 28006 Madrid (ES)
(74) Representative: Bernardo Noriega, Francisco

(57) **Abstract**

The invention provides highly functionalised spiro-fused azetidinones of formula I: 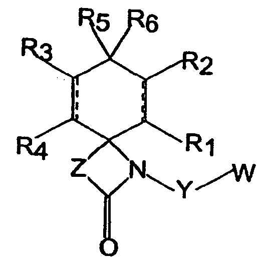 having a cyclohexane moiety with the desired number of protected or unprotected hydroxyl groups or carbonated structures, which are introduced with high stereo and regioselectivity, as well as processes for their obtention.

## Description

### FIELD OF THE INVENTION

The present invention relates to new regioselectively hydroxylated, protected and functionalized spirolactams and to processes for their synthesis.

### BACKGROUND OF THE INVENTION

Lactams are compounds of high interest due to their biological activities, for example well known β-lactams such as some penicillins, cephalosporins and carbapenems have antibacterial activity.

Spirolactams are one particular class of lactams that have shown interesting biological properties. Some spiro-fused azetidinones have been described as having antibacterial activity, see US 4,680,388, or hypocholesterolemic properties, see for example WO 94 17038. Additionally, if these compounds have the adequate functionality they are valuable intermediates towards different families of compounds. The spirolactam ring is the equivalent of an alpha amino or hydroxy aminoacid and opens many possibilities in diastero and/or enantioselective synthesis.

Miyazawa, E. et al. in *Heterocycles,* vol 59, 1:149-160 "Synthesis of spiro-fused nitrogen heterocyclic compounds via N-methoxy-N-acylnitrenium ions using phenyliodine (III) bis(trifluoroacetate) in trifluoroethanol" describe a process to obtain functionalised spirolactams including some spirodienones.

Kukugawa, Y. e al. in *J. Org. Chem.* 2003, vol. 68, 6739-6744 "Intramolecular cyclization with nitrenium ions generated by treatment of N-acylaminophthalimides with hypervalent iodine compounds: formation of lactams and spirofused lactams" describes the formation of functionalised spirolactams having diene and dienone funcionalities.

The conduritols, aminoconduritols, aminoinositols and their derivatives also possess interesting biological properties, some of them have been shown as being antitumoral and antibiotic. Although some synthetic processes exist for these compounds (See Yong-Uk Kwon et al, *J. Org. Chem*. 2002, vl. 67, 3327-3338 "Facile syntheses of all possible diastereomers of conduritol and various derivatives of inositol stereoisomers in high enantiopurity from *myo*-inositol"), there are still difficulties to obtain these compounds or corresponding analogues.

As it is apparent from the above, any efficient process for producing functionalised spirolactam compounds in high yield, with various functionalities, introduced in a controlled and regioselective manner, would be a wellcome contribution to the art and will open the door to a variety of biologically active compounds.

### SUMMARY OF THE INVENTION

Starting from the compounds described in our applications EP 04380104.2 and EP04076477.1, we found a basic set of processes that allows the controlled obtention of very stable, highly functionalised, spiro-fused azetidinones which are useful as intermediate compounds in the preparation of a variety of chemical structures, including, if necessary, by means of chimio-, loco-, regio-, diastero- and/or enantioselective processes. Additional carbon structures can be incorporated at the desired positions by means of simple reactions, generating new intermediates of interest.

In one aspect the invention provides a compound of formula I: 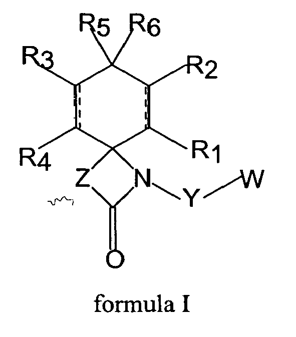 wherein R₁ and R₂ are each independently selected from H, OH or OPR;
R₃ and R₄ are each independently selected from H, OH, OPR, =O, cyano, substituted or unsubstituted alkyl, substituted or unsubstituted aryl, substituted or unsubstituted heterocyclyl, substituted or unsubstituted alkoxy, substituted or unsubstituted aryloxy, substituted or unsubstituted amino or halogen,
R₅ and R₆ together are =O or -O-(CH₂)ₘ-, wherein m is selected from 1, 2, 3, 4 or 5;
or R₅ is selected from H, OH, OPR and R₆ is selected from hydrogen, cyano, substituted or unsubstituted alkyl, substituted or unsubstituted cycloalkyl, substituted or unsubstituted alkenyl, substituted or unsubstituted alkinyl, substituted or unsubstituted aryl, substituted or unsubstituted heterocyclyl,
with the proviso that at least one of R₁, R₂, R₃, R₄ or R₅ is OH or OPR;
PR is an hydroxyl protecting group which can be the same or different on each of R₁, R₂, R₃, R₄ or R₅;
the dotted line represents a single or double bond, with the proviso that when both R₁ and R₂ or R₃ and R₄ are H then there is a double bond between the two C to which the H are linked;
Z is -(CRaRb)ₙ- wherein n is a number selected from 1, 2 or 3 and Ra and Rb are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino or halogen;
Y is selected from -O-, -S-, -N(RaRb)- or -C(O)-, wherein Ra and Rb are as previously defined and do not form a cyclic ring;
W is a group selected from substituted or unsubstituted arylalkyl, substituted or unsubstituted heterocyclylalkyl, substituted or unsubstituted alkenyl;
or a salt, complex or solvate thereof.

In one embodiment we prefer that n is 1. In this case Z is preferably -CHRa-.In another embodiment W is arylalkyl, preferably benzyl. In a further embodiment Y is preferably -O-.

The invention also provides for a process for the preparation of a compound according of formula I, which comprises in any order one or more of a step selected from the group consisting of:
a) hydroxylation or dihydroxylation
b) hydroxyl or carbonyl protection
c) nucleophilic attack at the carbonyl group or double bonds
d) hydroxyl inversion
e) allylic rearrangements
   applied to a compound of formula IV: 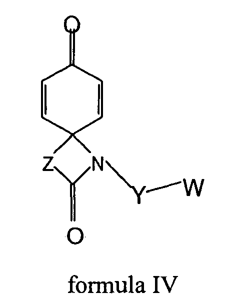 wherein Z , Y and W are as defined above.

### DETAILED DESCRIPTION OF THE INVENTION

The invention provides compounds of formula I as above defined. In the compounds of formula I, the group Z gives rise to a ring of 4, 5 or 6 members. Substitution on position Z creates a stereogenic center that could induce selective functionalisation on the benzodienone moiety. In a preferred embodiment Z is -(CHRa)ₙ-, the stereogenic center allows for the selectivity or specificity of any further reaction. Although rings of 5 or 6 are also comprised within the scope of the invention, in one embodiment the β-lactam ring (n= 1) is preferred because of the further uses that can be given to such compounds.

The group Y in the compounds of formula I plays a role in the stability and conformation. In an embodiment Y is preferably -O-, although other atoms are not excluded as long as the final product is stable.

The W group is important for the stabilization of the compound of formula I. Preferably it comprises unsaturated bonds or aromatic groups to increase the p interaction between W and the double bonds. Aralkyl groups and alkenyl groups are preferred since they give the best stability. In a particular embodiment, W is -CRaRb-Q or -SiRaRb-Q since the stability of the conformation is further improved by the presence of a -CRaRb-or a -SiRaRb- linker between Y and the substituent Q which has p (pi) interactions with the benzodienone moiety. The linker is preferably -CHRa-. This will advantageously open the way to diastero- and/or enantioselective synthesis in addition to the selection for one face which is explained below. Depending on the size of Ra it can also modulate the p (pi) interactions.

In one embodiment W is an aralkyl group. Among the aryl groups susbtituted or unsubstituted phenyl and naphthyl are preferred. Heterocyclylalkyl groups are also envisaged. Phenyl is the simplest subtituent and gives good results.

In the above definition of compounds of formula (I) and in the description the following terms have the meaning indicated:
"Alkyl" refers to a straight or branched hydrocarbon chain radical consisting of carbon and hydrogen atoms, containing no saturation, having 1-12, preferably one to eight carbon atoms, and which is attached to the rest of the molecule by a single bond, e. g., methyl, ethyl, n-propyl, i-propyl, n-butyl, t-butyl, n-pentyl, etc. Alkyl radicals may be optionally substituted by one or more substituents such as halo, hydroxy, alkoxy, carboxy, cyano, carbonyl, acyl, alkoxycarbonyl, amino, nitro, mercapto and alkylthio.
"Alkoxy" refers to a radical of the formula -ORa where Ra is an alkyl radical as defined above, e. g., methoxy, ethoxy, propoxy, etc. "Aryloxy" refers to a radical of formula -ORb wherein Rb is an aryl radical as defined below.
"Amino" refers to a radical of the formula-NH₂, -NHRa, -NRaRb.
"Aryl" refers to an aromatic hydrocarbon radical such as phenyl, naphthyl or anthracyl. The aryl radical may be optionally substituted by one or more substituents such as hydroxy, mercapto, halo, alkyl, phenyl, alkoxy, haloalkyl, nitro, cyano, dialkylamino, aminoalkyl, acyl and alkoxycarbonyl, as defined herein.
"Aralkyl" refers to an aryl group linked to an alkyl group such as benzyl and phenethyl.
"Cycloalkyl" refers to a saturated carbocyclic ring having from 3 to 8 carbon atoms.
"Heterocyclyl" refers to a stable 3- to 15- membered ring which consists of carbon atoms and from one to five heteroatoms selected from the group consisting of nitrogen, oxygen, and sulfur, preferably a 4-to 8-membered ring with one or more heteroatoms, more preferably a 5-or 6-membered ring with one or more heteroatoms. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quatemized; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran.
"Hydroxyl protecting group" refers to a group that blocks the OH function for further reactions and can be removed under controlled conditions. The hydroxyl protecting groups are well known in the art, representative protecting groups are silyl ethers such as trimethylsilyl ether, triethylsilyl ether, tert-butyldimethylsilyl ether, tert-butyldiphenylsilyl ether, tri-isopropylsilyl ether, diethylisopropylsilyl ether, thexyldimethylsilyl ether, triphenylsilyl ether, di-tert-butylmethylsilyl ether; alkyl ethers such as methyl ether, tert-butyl ether, benzyl ether, p-methoxybenzyl ether , 3,4-dimethoxybenzyl ether, trityl ether; allyl ether; alkoxymethyl ether such as methoxymethyl ether, 2-methoxyethoxymethyl ether, benzyloxymethyl ether, p-methoxybenzyloxymethyl ether, 2-(trimethylsilyl)ethoxymethyl ether; tetrahydropyranyl and related ethers; methylthiomethyl ether; Esters such as acetate ester, benzoate ester; pivalate ester; methoxyacetate ester; chloroacetate ester; levulinate ester; Carbonates such as benzyl carbonate, p-nitrobenzyl carbonate, tert-butyl carbonate, 2,2,2-trichloroethyl carbonate, 2-(trimethylsilyl)ethyl carbonate, allyl carbonate. Additional examples of hydroxyl protecting groups can be found in reference books such as Greene and Wuts' "Protective Groups in Organic Synthesis", John Wiley & Sons, Inc., New York, 1999.

References herein to substituted groups in the compounds of the present invention refer to the specified moiety that may be substituted at one or more available positions by one or more suitable groups, e. g., halogen such as fluoro, chloro, bromo and iodo ; cyano; hydroxyl ; nitro ; azido ; alkanoyl such as a C1-6 alkanoyl group such as acyl and the like ; carboxamido ; alkyl groups including those groups having 1 to about 12 carbon atoms or from 1 to about 6 carbon atoms and more preferably 1-3 carbon atoms ; alkenyl and alkynyl groups including groups having one or more unsaturated linkages and from 2 to about 12 carbon or from 2 to about 6 carbon atoms ; alkoxy groups having one or more oxygen linkages and from 1 to about 12 carbon atoms or 1 to about 6 carbon atoms ; aryloxy such as phenoxy ; alkylthio groups including those moieties having one or more thioether linkages and from 1 to about 12 carbon atoms or from 1 to about 6 carbon atoms ; alkylsulfinyl groups including those moieties having one or more sulfinyl linkages and from 1 to about 12 carbon atoms or from 1 to about 6 carbon atoms ; alkylsulfonyl groups including those moieties having one or more sulfonyl linkages and from 1 to about 12 carbon atoms or from 1 to about 6 carbon atoms ; aminoalkyl groups such as groups having one or more N atoms and from 1 to about 12 carbon atoms or from 1 to about 6 carbon atoms ; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

In our copending application, EP04380104.2, which is incorporated herein by reference in its entirety, we describe new compounds having a formula IV and processes for their obtention: 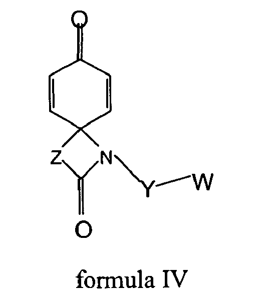 wherein Z and Y are as above defined and W is a group with sufficient electronic density to stabilize the compound through p (pi) interactions with the benzodienone moiety, preferably a group having unsaturated bonds or aromatic groups, more preferably it is selected from substituted or unsubstituted arylalkyl, substituted or unsubstituted heterocyclylalkyl, or substituted or unsubstituted alkenyl.

These compounds are remarkably stable due to p interactions between the W group and the benzodienone moiety. Additionally these compounds adopt a preferential conformation in which the W group blocks one of the faces of the benzodienone (hereinafter the β face) and is "fixed" there by the p interactions, directing further reactions to the free face of the benzodienone moeity (hereinafter the a face).

Taking advantage of this, we have found that starting from these compounds it is possible to regioselectively hydroxylate, functionalise and protect the different positions of the benzodienone group, to give a broad range of stable compounds having the desired functionality and protection at each of the 5 available positions. Further, carbonated substituents can be introduced on the obtained compounds, to generate a carbon skeleton at wish. All these highly functionalised compounds are useful as building blocks for a wide variety of bioactive compounds.

In our copending EP04076477.1 we describe the dihydroxylation that takes place regioselectively, only via the a face:

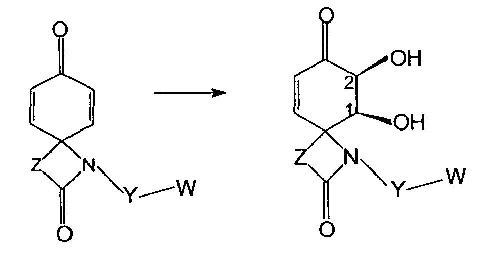

This oxidation occurs readily under mild conditions, such as using OsO₄ in a polar solvent, for example a mixture of water an ketone, in the presence of an amine such as N-methylmorpholine *N*-oxide. Alternative oxidation systems will be readily apparent to the person skilled in the art and can be found in standard references for organic synthesis such as Noyori, R. "Asymmetric catalysis in organic synthesis", John Wiley and Sons, Inc. (1994) or Ojima, I. "Catalytic asymmetric synthesis VCH, (1993).

The dihydroxylated compound can be selectively protected. Indeed, when carrying out a protection such as with C1-TBDMS we found that the hydroxyl at position 6 reacted until being completely protected, and only then the OH at position 1 is protected.

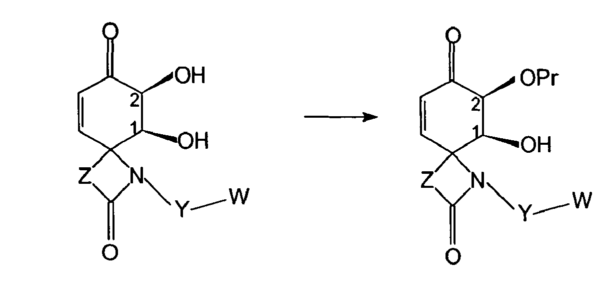

Without being bound by theory, we believe that this is due to the existence of C-H p interactions between the H at position 6 and the W group. This means that the -OH at position 6 is in an equatorial conformation, more reactive, while the -OH at position 5 is in an axial conformation, less reactive. This allows the selective reaction of one position with respect to the other.

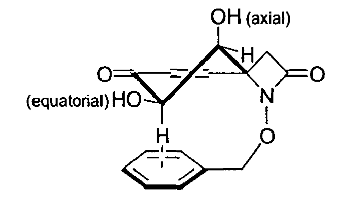

Therefore, both the facial selection (a versus β) when carrying out the hydroxylation, and the different reactivity of positions 5 and 6, due to the particular conformations generated by the presence of the W group and its interactions with the rest of the molecule, allows for a fine tuned control of the functionalisation of the molecule.

The two hydroxy groups can be protected with the same protecting group as explained above, or with different protecting groups, first protecting the position 6 and then the position 5:

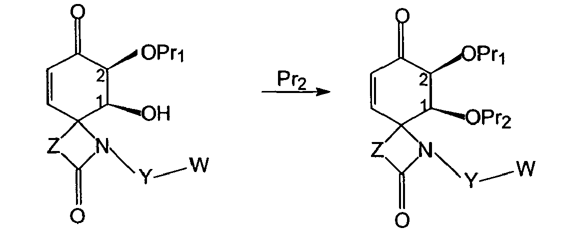

To introduce the hydroxyl protecting groups standard procedures can be used, such as those described in Greene and Wuts' "Protective Groups in Organic Synthesis", John Wiley & Sons, Inc., New York, 1999 or Kocienski, P.J. "Protecting Groups", 3^{rd} Ed.Thieme Chemistry, 2003.

In another embodiment the carbonyl group can also be selectively functionalized for example by Nucleophilic addition. Importantly, the lactam group does not react instead because it has a Weinreb type of amide. Thus cyanides, organolithium compounds, Grignard's reagents, ketones among other can be easily added to introduce the desired functionality at this position. If an hydride is used then an hydroxy at position 3 is generated. Suitable procedures for this kind of reactions are known in the art and can be found for example in Fischer, A. et al *J. Org. Chem*, 1987, 52, 4464-4468 "Formation of 4-nitrocyclohexa-2,5-dienols by addition of organolithium reagents to 4-alkyl-4-nitrocyclohexa-2,5-dione"; Wipf et al., *Angew. Chem. Int. Ed. Engl*. 1997, 36, no.7, 764-767; Fischer, A. et al., *Tetrahedron lett.,* 1980, 21, 701-704; Carreno, M. et al., *J. Org. Chem*, 1997, 62, 26, 9128-9137.

The additions can be done independently of the functionalisation of the other positions. We have found that when the compound of formula IV is first dihydroxylated and then the addition to the carbonyl is carried out, complete stereoselectivity is achieved.

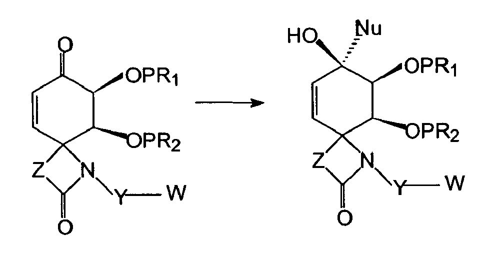

Depending on the Nucleophilic reagent used the product of the reaction can have different structures. For example, if an epoxide with a defined conformation is desired, then a reagent such as an S ylide can be used. Under conditions of a Corey's epoxidation only one epoxide is obtained [a) E. J. Corey; Michael Chaykovsky J. Am. Chem. Soc. 87, 1965, 1353-1364. b) Steven P. Tanis, Mark C. McMills, Paul M. Herrinton J. Org. Chem. 50, 1985, 5887-5889. c) Malcolm Chandler, Richard Conroy, Anthony W. J. Cooper, R. Brian Lamont, Jan J. Scicinski, James E. Smart, Richard Storer, Niall G. Weir, Richard D. Wilson and Paul G. Wyatt J. Chem. Soc. Perkin Trans. 1, 1995, 1189-1197]:

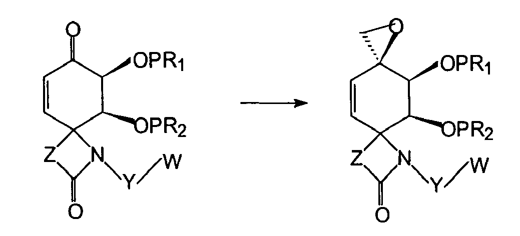

Alternatively a double bond can be generated, for example under Wittig's conditions. In this case the Nucleophile is a C ylide.

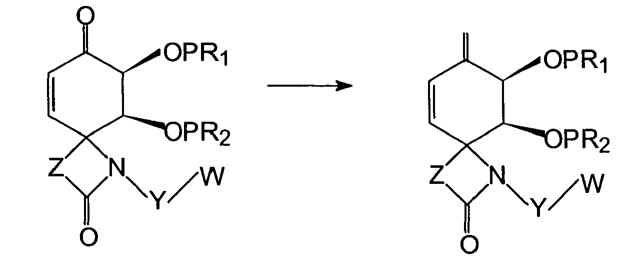

The double bond can have further substituents at position 10 depending on the reagent used. From this structure, with two differentiated double bonds, further reactions can be carried out as we will explain below.

If the Nucleophile is directed to the double bond instead of the carbonyl then the functionality or the additional carbon group is added at position 9:

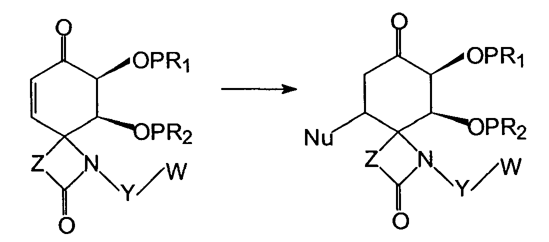

In such a way for example cyanide or an allylic group can be introduced at position 9, opening new possibilities for further construction. It will be understood that once this Nu is introduced, then the above reactions on the carbonyl group, such as another nucleophilic attack can be done to generate new structures. For example, if Corey's epoxidation or Wittig's reaction are carried out on this new substrate, the following structures can be obtained:

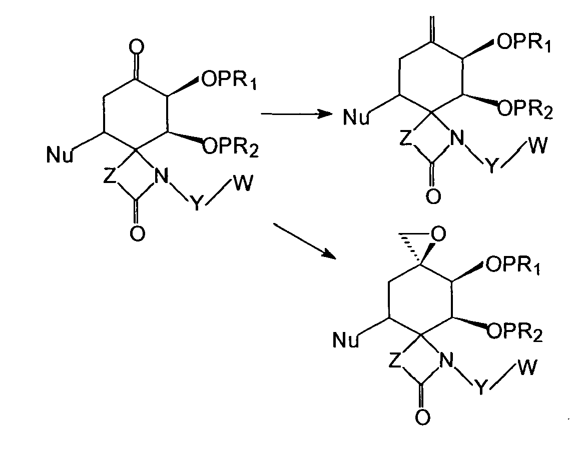

The epoxides can be opened to give an hydroxyl group at position 10. This can be done with the simultaneous introduction of a Nucleophilic group at position 9:

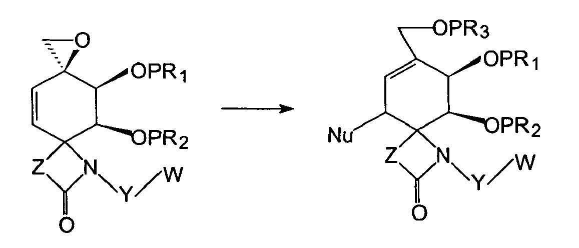

In this way we generate a new double bond between positions 7 and 8 which again opens many possibilities for further functionalisation. For example, a simple dihydroxylation will generate a highly functionalised structure, with two new and differentiated hydroxy groups:

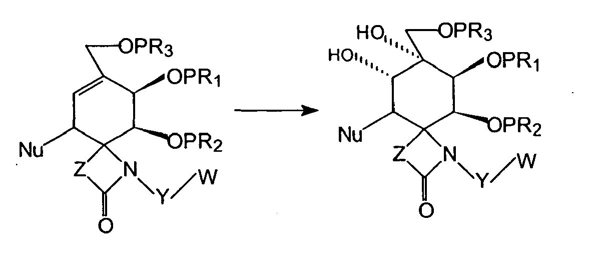

Under mild conditions (for example OsO₄ as descibed above) the initial dihydroxy compound can be hdroxylated again to generate a structure with 4 hydroxy groups: 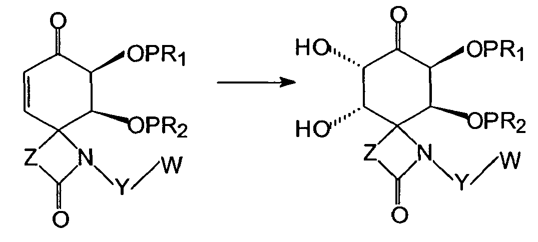 If desired, the stereochemistry of the free hydroxy groups can be subjected to inversions. It will be readily apparent to the person skilled in the art that these rae suitable precursor to inositols and conduritols , through opening of the lactame ring and removing, latter on, their acetate portion by processes that envolve retroaldolic or retro-Staudinger like reactions. Other analogues of these valuable biological compounds can be prepared if we start from the different structures as we are describing here.

The dihydroxylation can be carried out on the product of Wittig's reaction, in this case two differentiated hydroxy groups are introduced at positions 7 and 10:

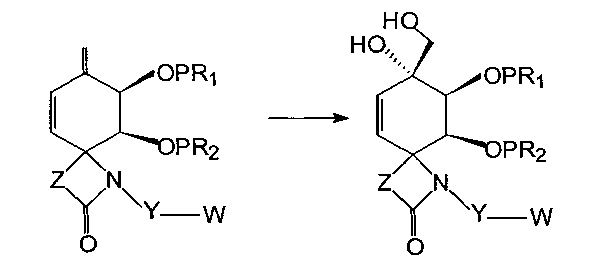

If instead of a dihydroxylation we do an epoxidation with, for example, a peroxide under mild conditions, we obtain an epoxide with just the opposite configuration at 7 when compared with the epoxide obtained directly under Corey's conditions:

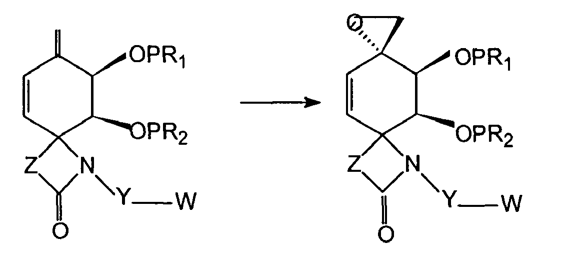

Thus, even through all these reactions we are still able to control the stereochemistry at the different positions. This allows us to prepare intermediates with the adequate configuration in order to reach downstream the different stereoisomers of biologically active compounds.

The presence of vicinal hydroxyl groups allows the simultaneous protection of two of them through the use of diol protecting groups if desired. Among the diol protecting groups that can be used we have O,O-acetals such as isopropylidene acetals (acetonides); cyclohexylidene and cyclopentylidene acetals; arylmethylene acetals; methylene acetals; diphenylmethylene acetals; 1,2-diacetals such as dispiroketal (dispoke) derivatives, cyclohexane-1,2-diacetals, butane-2,3-diacetals; silylene derivatives; 1,1,3,3-tetraisopropyldisiloxanylidene derivatives or N,O-acetals. Additional examples of diol protecting groups can be found in reference books such as Greene and Wuts' "Protective Groups in Organic Synthesis", John Wiley & Sons, Inc., New York, 1999. Additionally, borolanes can be formed on the two vicinal hydroxy groups, for example using phenylboric acid.

For example, dihydroxylation and protection can be carried out simultaneously: 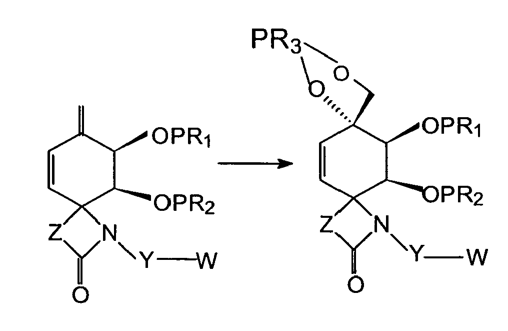 Or, if desired, only one of the hydroxy groups can be selectively protected due to their different nature (primary versus tertiary alcohol):

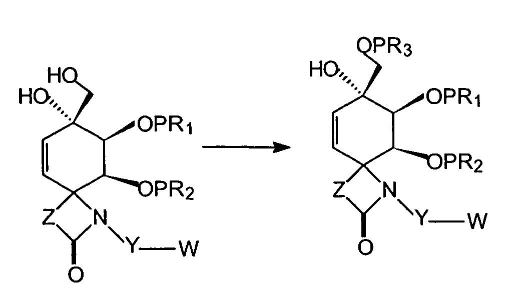

A carbolactone can be produced through reaction of a functionalised Nucleophile at the carbonyl group and lactonization with the unprotected hydroxyl group which is present at position 8, for example using methyl (triphenylphosphoranylidene)acetate:

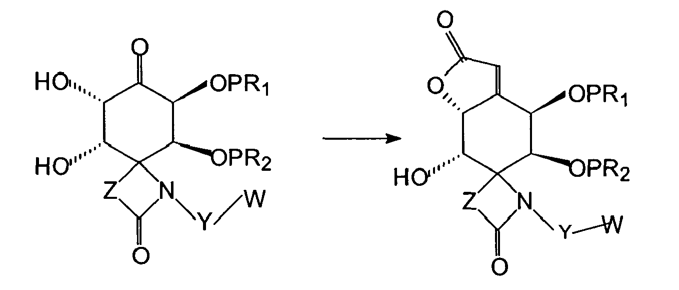

Or, under stronger conditions: 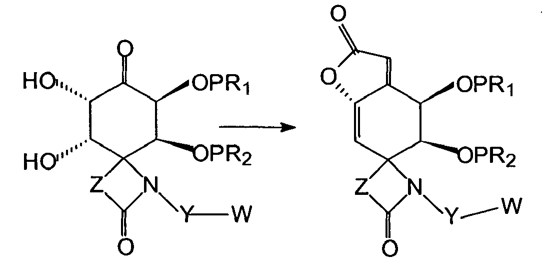 Again, we obtain a new structure plenty of functionalities with can be further subjected to additional reactions.

As can be understood from the above and will be apparent to the person skilled in the art, due to the particular conformation and reactivity characteristics of the described compounds, a great number of possibilities can be achieved. It is important to point out that the obtained compounds of formula I will present a carefully crafted functionality at the different positions 5, 6, 7, 8, 9 and 10, and the desired stereochemistry. The same structure can be reached through different combinations of the basic reactions. The introduction of different protecting groups opens the route to very selective further reactions by chosing the appropriate protection-deprotection strategies.

The process to obtain any of these compounds can be readily designed by starting from a compound of formula IV above and then applying a basic set of reactions selected from:
a) Hydroxilation or dihydroxylation: as above explained, using mild (such as OsO₄/ N oxide amine) or strong systems (such as RuCl₃) depending on the position to be hydroxylated. Alternative systems are also envisaged.
b) Nucleophilic attack at the carbonyl group: for example with a carbanion on a sp, sp2 or sp3 C, the carbanion can be prepared or generated in situ; or with an hydride. The Nucleophilic attack allows the introduction of new functionalities, new carbonated structures and also epoxides or double bonds, depending on the reagents used.
c) Hydroxyl inversion : for example through epimerization or inversion, for example in Mitsunobu conditions.
d) Hydroxyl or carbonyl protection: as explained above, using the same or different protecting groups in conditions as explained above.
e) Allylic rearrangements: allows migration of a double bond.

Although each of these procedures is well known and the appropriate reagents can be selected by the person skilled in the art of organic synthesis, for example from those given in the references above, their application to the our structures gives unexpected results in terms of reactivity and selectivity.

The following scheme I illustrates some of these possibilities:

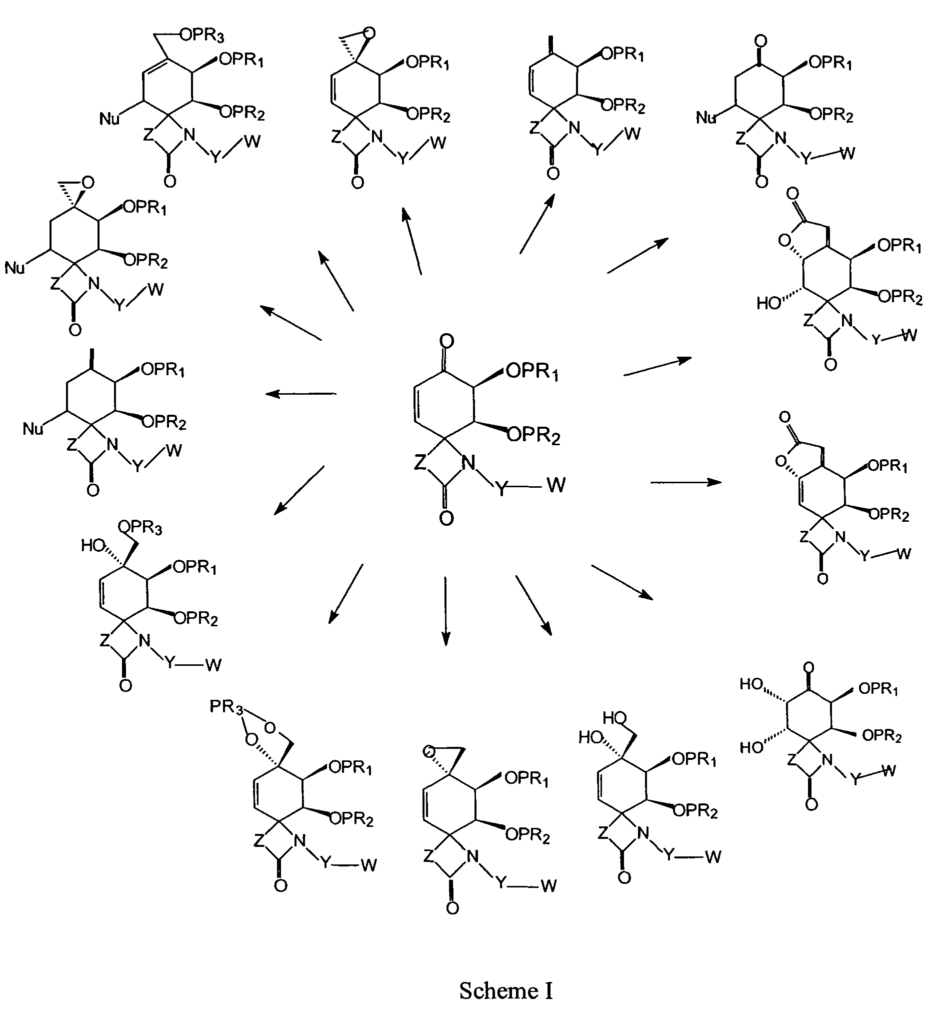

All the compounds will be obtained as racemic mixtures. However, if enantiopurity is desired, this can be achieved by introducing a chiral center in the W group as explained above, or using chiral reagents or catalysts. Therefore the compounds of the present invention represented by the above described formula (I) may include pure enantiomers depending on the presence of stereogenic centers or diastereoisomers. The single isomers, enantiomers or diastereoisomers and mixtures thereof fall within the scope of the present invention. Mixtures of different diasteroisomers can be separated by conventional techniques.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The compounds of the invention may be in crystalline form either as free compounds or as solvates (e.g. hydrates) and it is intended that both forms are within the scope of the present invention. Methods of solvation are generally known within the art.

The invention will be further illustrated by means of examples, which should not be interpreted as limiting the scope of the claims.

### EXAMPLES

### General Methods and Materials.

All reactions described below were carried out under argon atmosphere unless otherwise noted. The solvents used were distilled and dried under argon atmosphere before use. All starting materials were purchased commercially (Aldrich, Fluka and Merck) and used without further purification. Flash Chromatography was executed on columns loaded with 230-400 mesh silica gel Merck. TLC was carried out on silica gel Merck (Kieselgel 60F-254).

Melting points (mp) were determined on a Reichert Microscopic Hot-Stage and are uncorrected. ¹H and ¹³C NMR spectra were measured on a Varian Gemini-200 and a Varian Inova-300 spectrometer with (CH₃)₄Si as an internal reference and CDCl₃ as solvent unless otherwise noted. Both ¹H and ¹³C NMR spectral data are reported in parts per million (δ) relative to residual sign of the solvent (CDCl₃, 7.26 ppm and 77.0 ppm for ¹H and ¹³C NMR, respectively). ¹H and ¹³C NMR designations are: s (singlete); br. s (broad singlete); d (doublete); br. d (broad doublete); t (triplete); q (quartete); m (multiplete). Infrared (IR) spectra were record on a Perkin-Elmer FT-IR spectrometer. UV spectra were record on a Perkin-Elmer 402 spectrometer. Low-resolution mass (LRMS) spectra were obtained on a Hewlett Packard 5973 MSD spectrometer with a direct inlet system (EI) at 70 eV. Microanalytical data (E.A.) were obtained on a Perkin-Elmer 240C and Heraus CHN-O instruments at the Instrumental Analysis Department of Instituto de Quimica Orgánica General (C.S.I.C.).

The compounds of general formula I and II were nominated as derivatives of 1-azaspiro[3.5]nonan-2-one and were numerated as described below.

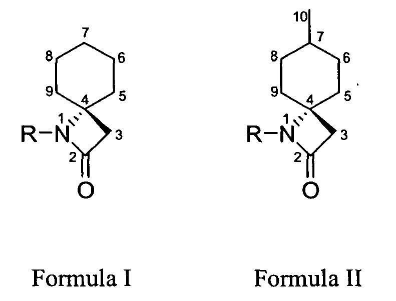

### Example 1

### Preparation of rac-(4R,5S,6S,7S)-1-benzyloxy-6-tert-butyldimethylsilyloxy-7,10-epoxy-7-methyl-5-trimethylsilyloxy-1-azaspiro[3.5]nona-8-en-2-one (2)

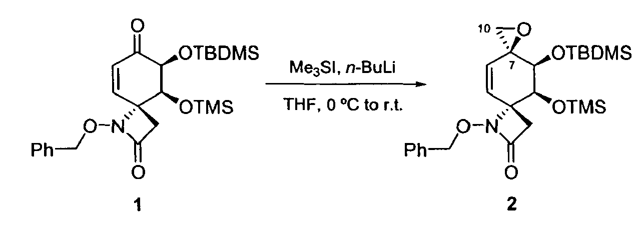

To a stirred suspension of powdered trimethylsulfonium iodide (52 mg, 0.252 mmol) in THF (0.9 ml) was added dropwise at 0 °C n-butyl lithium (0.16 ml, 1.6 M solution in hexane, 0.252 mmol). After stirring of the mixture for 15 min at this temperature, only a slight precipitate remained; a solution of *rac*-(4*R*,5*S*,6*S*)-1-benzyloxy-6-*tert-*butyldimethylsilyloxy-5-trimethylsilyloxy-1-azaspiro[3.5]nona-8-en-2,7-dione (1) (100 mg, 0.210 mmol) in THF (0.6 ml) was added dropwise. The mixture was stirred for 30 min at 0 °C, and then for 2 h at room temperature. The reaction was quenched with Na₂HPO₄ 0.1 M buffer (2.5 ml) and AcOEt (2.5 ml). The layers were separated and aqueous phase was extracted with AcOEt (3 x 5 ml). The combined extracts were dried over Na₂SO₄, filtered and concentrated under reduced pressure. The residue was purified by silica gel column chromatography (hexane-AcOEt, 8:1) to give *rac*-(4*R*,5*S*,6*S*,7*S*)-1-benzyloxy-6-*tert-*butyldimethylsilyloxy-7,10-epoxy-7-methyl-5-trimethylsilyloxy-1-azaspiro[3.5]nona-8-en-2-one (2) as a low melting point white solid (84 mg, 82%).
***R***_{***f***} = 0.50 (TLC, hexane-AcOEt, 3:1); yield, 82%; white solid; ^{**1**}**H-NMR** (300MHz, CDCl₃): δ 7.34 (5H, m, Ph), 5.52 (1H, d, J = 10.1 Hz, H-9), 5.06 (1H, dd, J = 10.1, 1.9 Hz, H-8), 5.00 (1H, *part A syst. AB,* J = 11.5 Hz, OCH₂Ph), 4.84 (1H, *part B syst. AB,* J = 11.5 Hz, OCH₂Ph), 4.17 (1 H, d, J = 1.9 Hz, H-6), 3.50 (1 H, t, J = 1.9 Hz, H-5), 3.43 (1H, *part A syst. AB,* J = 13.9 Hz, H-3), 2.87 (1H, *part A syst. AB,* J = 5.0 Hz, H-10), 2.73 (1H, *part B syst. AB,* J = 5.0 Hz, H-10), 2.34 (1H, *part B syst. AB,* J = 13.9 Hz, H-3), 0.83 (9H, s, C(CH₃)₃), 0.14 (9H, s, Si(CH₃)₃), 0.04 (3H, s, SiCH₃), 0.02 (3H, s, SiCH₃); ^{**13**}**C-NMR** (75MHz, CDCl₃): δ 165.9, 135.7, 133.1, 129.6, 128.9, 128.6, 128.4, 78.7, 77.4, 68.7, 66.3, 58.9, 53.0, 40.7, 25.7, 18.2, 0.2, -4.9, -5.0 ; **IR** (NaCl,CCl₄): v 3028, 2956, 2857, 1778, 1472, 1410, 1253, 1152, 1116, 987, 930, 879, 839, 778 cm⁻¹; **LRMS(API-ES**^{**+**}**):** m/z 1001(2M+Na)⁺, 512(M+Na)⁺, 490(M+H)⁺.

### Example 2

### Preparation of rac-(4R,5S,6S)-1-benzyloxy-6-tert-butyldimethylsilyloxy-7-methylen-5-trimethylsilyloxy-1-azaspiro[3.5]nona-8-en-2-one (3)

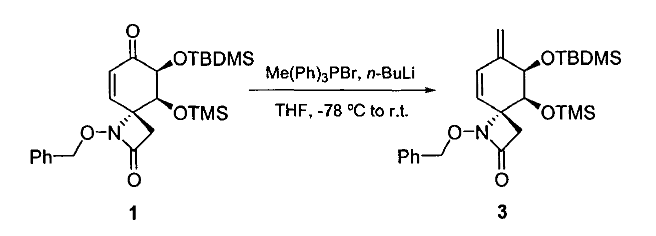

To a stirred suspension of methyltriphenylphosphonium (292 mg, 0.802 mmol) in THF (7.5 ml) was added dropwise at -78 °C n-butyl lithium (0.5 ml, 1.6 M solution in hexane, 0.802 mmol) and the resulting light yellow solution was stirred for 15 min at this temperature. Then, a solution of *rac*-(4*R*,5*S*,6*S*)-1-benzyloxy-6-*tert*-butyldimethylsilyloxy-5-trimethylsilyloxy-1-azaspiro[3.5]nona-8-en-2,7-dione (1) (381 mg, 0.802 mmol) in THF (7.5 ml) was added dropwise and the mixture was stirred for 1 h at room temperature. The reaction was quenched at 0 °C with H₂O (5 ml), the layers were separated and aqueous phase was extracted with AcOEt (3 x 10 ml). The combined extracts were dried over MgSO₄, filtered and concentrated under reduced pressure. The residue was purified by silica gel column chromatography (hexane-AcOEt, 8:1) to give *rac*-(4*R*,5*S*,6*S*)-1-benzyloxy-6-*tert*-butyldimethylsilyloxy-7-methylen-5-trimethylsilyloxy-1-azaspiro[3.5]nona-8-en-2-one (3) as a colourless oil (257 mg, 68%).
***R***_{***f***} = 0.58 (TLC, hexane-AcOEt, 3:1); **yield,** 68%; colourless oil; ^{**1**}**H-NMR** (300MHz, CDCl₃): δ 7.34 (5H, m, Ph), 5.97 (1H, *part A syst. AB,* J = 13.8 Hz, H-9), 5.38 (1H, *part B syst. AB*, J = 13.8 Hz, H-8), 5.01 (2H, br. s, CH₂=C), 4.98 (1H, *part A syst. AB*, J = 11.7 Hz, OCH₂Ph), 4.87 (1H, *part B syst. AB*, J = 11.7 Hz, OCH₂Ph), 4.28 (1H, br. s, H-6), 4.06 (1H, br. s, H-5), 3.47 (1H, *part A syst. AB*, J = 14.1 Hz, H-3), 2.28 (1H, *part B syst. AB,* J = 14.1, H-3), 0.80 (9H, s, C(CH₃)₃), 0.15 (9H, s, Si(CH₃)₃), 0.07 (3H, s, SiCH₃), -0.01 (3H, s, SiCH₃); ^{**13**}**C-NMR** (75MHz, CDCl₃): δ 166.0, 144.3, 135.6, 130.6, 129.3,128.8, 128.6, 128.4, 115.6, 78.6, 76.1, 69.2, 66.6, 40.3, 25.6, 18.0, 0.3, -4.6, -4.7; **IR** (NaCl,CCl₄): ν 3028, 2956, 2927, 2855, 1779, 1656, 1611, 1472, 1367, 1252, 1138, 1101, 988, 883, 840 cm⁻¹; **LRMS(API-ES**^{**+**}**)**: m/z 969(2M+Na)⁺, 496(M+Na)⁺, 474(M+H)⁺.

### Example 3

### Preparation of rac-(4R,5S,6S,7R)-1-benzyloxy-6-tert-butyldimethylsilyloxy-7-hydroxy-7-hydroxymethyl-5-trimethylsilyloxy-1-azaspiro[3.5]nona-8-en-2-one (4)

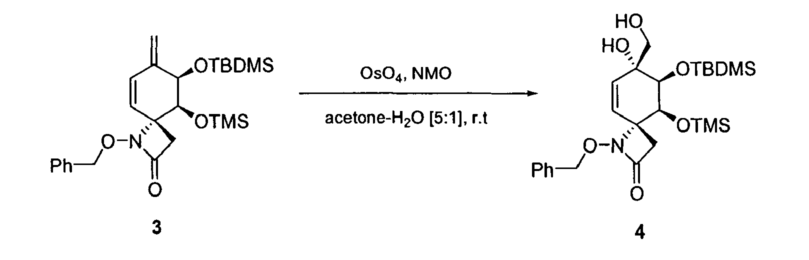

To a solution of *rac*-(4*R*,5*S*,6*S*)-1-benzyloxy-6-*tert*-butyldimethylsilyloxy-7-methylen-5-trimethylsilyloxy-1-azaspiro[3.5]nona-8-en-2-one (3) (235 mg, 0.496 mmol) in acetone (4 ml) was added sequentially at room temperature H₂O (0.8 ml), *N*-methylmorpholine *N*-oxide (132 mg, 1.091 mmol) and osmium tetroxide (0.37 ml, 2.5 wt. % solution in 2-methyl-2-propanol, 0.030 mmol). The resulting mixture was stirred at room temperature until the reaction was complete (16 h, TLC monitoring, hexane-AcOEt, 3:1), and then quenched with 10% aqueous Na₂S₂O₃ solution (1.5 ml). After 15 min, the mixture was extracted with AcOEt (3 x 4 ml). The combined organic extracts were dried over Na₂SO₄, filtered and concentrated under reduced pressure to give *rac*-(4*R*,5*S*,6*S*,7*R*)-1-benzyloxy-6-*tert-*butyldimethylsilyloxy-7-hydroxy-7-hydroxymethyl-5-trimethylsilyloxy-1-azaspiro[3.5]nona-8-en-2-one (4) as a low melting point pale yellow solid (240 mg, 95%).
***Rf** =* 0.13 (TLC, hexane-AcOEt, 3:1), 0.64 (TLC, hexane-AcOEt, 1:1); yield, 95%; pale yellow solid; ^{**1**}**H-NMR** (300MHz, CDCl₃): δ 7.41-7.32 (5H, m, Ph), 5.47 (1H, d, J = 9.9 Hz, H-9), 5.41 (1H, d, J = 9.9 Hz, H-8), 5.01 (1H, *part A syst. AB,* J = 11.5 Hz, OCH₂Ph), 4.84 (1H, *part B syst. AB,* J = 11.5 Hz, OCH₂Ph), 4.48 (1 H, br. s, H-6 or H-5), 3.83 (1 H, br. s, H-5 or H-6), 3.67 (1 H, br. d, J = 11.1 Hz, CH₂-OH), 3.45 (1 H, d, J = 11.1 Hz, CH₂₋OH), 3.24 (1 H, br. d, J = 12.4 Hz, H-3), 2.26 (1 H, br. d, J = 12.4 Hz, H-3), 2.04 (2H, s br., OH), 0.81 (9H, s, C(CH₃)₃), 0.19 (9H, s, Si(CH₃)₃), 0.13 (3H, s, SiCH₃), 0.08 (3H, s, SiCH₃); ^{**13**}**C-NMR** (75MHz, CDCl₃): δ 165.3, 133.4, 129.5, 129.2, 128.8, 128.6, 128.5, 78.8, 76.2, 73.7, 68.0, 66.5, 65.8, 40.2, 26.0, 18.1, 0.4, -2.8, -5.4; **IR** (NaCl,CCl₄): v 3434, 3028, 2956, 2858, 1755, 1478, 1462, 1456, 1411, 1389, 1362, 1253, 1141, 1090, 918, 882, 837, 756 cm⁻¹; **LRMS(API-ES**^{**+**}**):** m/z 1037(2M+Na)⁺, 530(M+Na)⁺, 508(M+H)⁺.

### Example 4

### Preparation of rac-(4R,5S,6S,9R)-1-benzyloxy-6-tert-butyldimethylsilyloxy-9-cyano-5-trimethylsilyloxy-1-azaspiro[3.5]nona-8-en-2,7-dione (5)

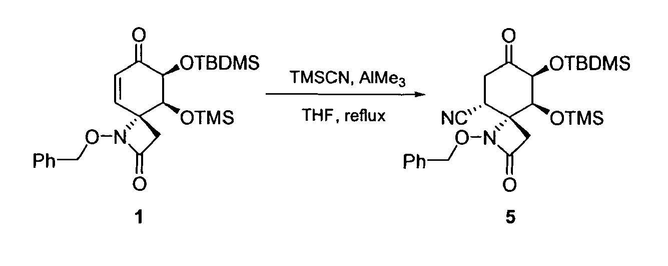

To a stirred solution of cyanotrimethyl silane (56 µl, 0.411 mmol) and trimethylaluminium (0.19 ml, 2.0 M solution in toluene, 0.374 mmol) in THF (0.5 ml) was added at room temperature a solution of *rac*-(4*R*,5*S*,6*S*)-1-benzyloxy-6-*tert-*butyldimethylsilyloxy-5-trimethylsilyloxy-1-azaspiro[3.5]nona-8-en-2,7-dione (1) (89 mg, 0.187 mmol) in THF (0.5 ml). The resulting mixture was stirred at reflux for 24 h and then concentrated under reduced pressure. The residue was dissolved in toluene (2 ml), was washed with cold NH₄Cl sat. (1 ml) and Na₂HPO₄ 0.1 M buffer (1 ml), dried over Na₂SO₄, filtered and concentrated under reduced pressure. The residue was purified by silica gel column chromatography (hexane-AcOEt, 5:1) to give *rac*-(4*R*,5*S*,6*S*,9*R*)-1-benzyloxy-6-*tert-*butyldimethylsilyloxy-9-cyano-5-trimethylsilyloxy-1-azaspiro[3.5]nona-8-en-2,7-dione (5) as a low melting point white solid (21 mg, 22%).
***R***_{***f***} = 0.41 (TLC, hexane-AcOEt, 3:1); yield, 22%; white solid; ^{**1**}**H-NMR** (300MHz, CDCl₃): δ 7.44-7.30 (5H, m, Ph), 5.31 (1H, *part A syst. AB,* J = 10.7 Hz, OCH₂Ph), 5.11 (1H, *part B syst. AB*, J = 10.7 Hz, OCH₂Ph), 4.71 (1H, d, J = 2.2 Hz, H-6), 4.13 (1H, d, J = 2.2 Hz, H-5), 3.66 (1H, dd, J = 13.4, 5.4 Hz, H-9), 2.92 (1H, *part A syst. AB*, J = 14.5 Hz, H-3), 2.76 (1H, *part B syst. AB,* J = 14.5 Hz, H-3), 2.63 (1H, dd, J = 13.4, 5.4 Hz, H-8), 2.21 (1H, t, J = 13.4 Hz, H-8), 0.85 (9H, s, C(CH₃)₃), 0.09 (9H, s, Si(CH₃)₃), 0.08 (3H, s, SiCH₃), -0.06 (3H, s, SiCH₃); ^{**13**}**C-NMR** (75MHz, CDCl₃): δ 201.0, 164.1, 133.9, 129.5, 129.4, 128.9, 117.1, 80.8, 78.9, 78.0, 67.3, 42.2, 38.3, 32.0, 25.8, 18.5, 0.5, -4.8, -5.6; **IR** (KBr): ν 3425, 2956, 2927, 2855, 2233, 1772, 1631, 1454, 1364, 1255, 1107, 1064, 841 cm⁻¹; **LRMS(API-ES**^{**+**}**)**: m/z 525(M+Na)⁺, 503(M+H)⁺.

### Example 5

### Preparation of rac-(4R,5S,6S,8S,9S)-1-benzyloxy-6-tert-butyldimethylsilyloxy-8,9-dihydroxy-5-trimethylsilyloxy-1-azaspiro[3.5]nonan-2,7-dione (6)

### Method A.

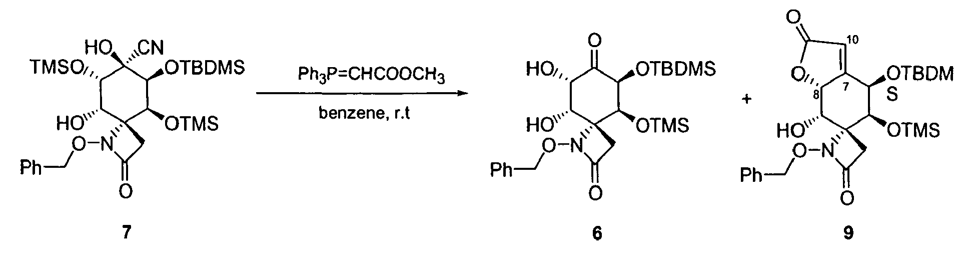

To a solution of *rac*-(4*R*,5*S*,6*S*,7*S*,8*S*,9*S*)-1-benzyloxy-6-*tert*-butyldimethylsilyloxy-7-cyano-7,9-dihydroxy-5,8-bis(trimethylsilyloxy)-1-azaspiro[3.5]nonan-2-one (7) (58 mg, 0.095 mmol) in benzene (1.0 ml) was added at room temperature a solution of methyl (triphenylphosphoranylidene)acetate (76 mg, 0.228 mmol) in benzene (2 ml). The mixture was stirred to reflux for 11 h. The reaction was quenched with Na₂HPO₄ 0.1M buffer (3 ml) and AcOEt (3 ml). The layers were separated and aqueous phase was extracted with AcOEt (3 x 6 ml). The combined extracts were dried over Na₂SO₄, filtered and concentrated under reduced pressure. The residue was purified by silica gel column chromatography (hexane-AcOEt, 4:1) to give *rac*-(4*R*,5*S*,6*S*,8*S*,9*S*)-1-benzyloxy-6-*tert-*butyldimethylsilyloxy-8,9-dihydroxy-5-trimethylsilyloxy-1-azaspiro[3.5]nonan-2,7-dione (6) as a white solid (19 mg, 38 %) and *rac*-(4*R*,5*S*,6*S*,8*S*,9*S*)-1-benzyloxy-6-*tert-*butyldimethylsilyloxy-9-hydroxy-7-methylen-5-trimethylsilyloxy-1-azaspiro[3.5]nonan-2-on-10,8-carbolactone (9) as a white solid (10 mg, 20 %).

### Method B.

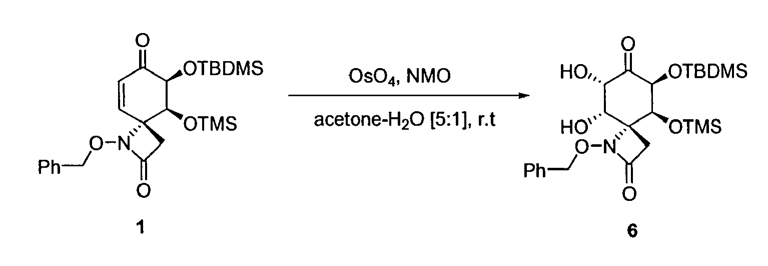

To a solution of *rac*-(4*R*,5*S*,6*S*)-1-benzyloxy-6-*tert*-butyldimethylsilyloxy-5-trimethylsilyloxy-1-azaspiro[3.5]nona-8-en-2,7-dione (1) (49 mg, 0.103 mmol) in acetone (0.5 ml) was added sequentially at room temperature H₂O (0.1 ml), *N*-methylmorpholine *N*-oxide (26 mg, 0.227 mmol) and osmium tetroxide (0.077 ml, 2.5 wt. % solution in 2-methyl-2-propanol, 0.006 mmol). The resulting mixture was stirred at room temperature until the reaction was complete (15 h, TLC monitoring, hexane-AcOEt, 1:1), and then quenched with 10% aqueous Na₂S₂O₃ solution (0.2 ml). After 20 min, the mixture was extracted with AcOEt (5 x 2 ml). The combined organic extracts were dried over Na₂SO₄, filtered and concentrated under reduced pressure. The residue was purified by silica gel column chromatography (hexane-AcOEt, 2:1) to give *rac*-(4*R*,5*S*,6*S*,8*S*,9*S*)-1-benzyloxy-6-*tert*-butyldimethylsilyloxy-8,9-dihydroxy-5-trimethylsilyloxy-1-azaspiro[3.5]nonan-2,7-dione (6) as a white solid (32 mg, 62%).
***Rf*** = 0.10 (TLC, hexane-AcOEt, 3:1), 0.55 (TLC, hexane-AcOEt, 1:1); **yield,** 62%; white solid; ^{**1**}**H-NMR** (300MHz, CDCl₃): δ 7.43-7.39 (2H, m, Ph), 7.34-7.32 (3H, m, Ph), 5.11 (1H, *part A syst. AB*, J = 10.1 Hz, OCH₂Ph), 5.05 (1H, *part B syst. AB*, J = 10.1 Hz, OCH₂Ph), 4.74 (1H, dd, J = 3.6, 3.4 Hz, H-8), 4.44 (1H, *part A syst. AB*, J = 3.2 Hz, H-6), 4.26 (1H, *part B syst. AB*, J = 3.2 Hz, H-5), 4.17 (1H, dd, J = 3.6, 1.6 Hz, H-9), 3.52 (1 H, d, J = 3.4 Hz, HO-C(8)), 3.46 (1H, *part A syst. AB*, J = 13.7 Hz, H-3), 2.74 (1H, s, HO-C(9)), 2.38 (1H, *part B syst. AB*, J = 13.7 Hz, H-3), 0.85 (9H, s, C(CH₃)₃), 0.12 (3H, s, SiCH₃), 0.08 (9H, s, Si(CH₃)₃), 0.00 (3H, s, SiCH₃); ¹³C-NMR (75MHz, CDCl₃): δ 205.7, 165.3, 135.2, 129.3, 129.0, 128.6, 128.3, 78.7, 77.8, 75.4, 72.1, 67.6, 66.9, 37.5, 25.5, 18.0, 0.3, -5.0, -5.2 ; **IR** (NaCl,CCl₄): ν 3435, 2955, 2891, 2858, 1761, 1740, 1471, 1253, 1154, 1139, 1109, 887, 884, 780 cm⁻¹; **LRMS(API-ES**^{**+**}**)**: m/z 1041(2M+Na)⁺, 532(M+Na)⁺, 510(M+H)⁺.

### Example 6

### Preparation of rac-(4R,5S,6S,7R)-1-benzyloxy-6-tert-butyldimethylsilyloxy-7,10-epoxy-7-methyl-5-trimethylsilyloxy-1-azaspiro[3.5]nona-8-en-2-one (8)

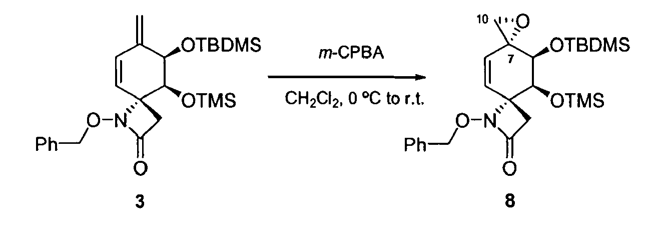

To a stirred solution of *rac*-(4*R*,5*S*,6*S*)-1-benzyloxy-6-*tert*-butyldimethylsilyloxy-7-methylen-5-trimethylsilyloxy-1-azaspiro[3.5]nona-8-en-2-one (3) (200 mg, 0.422 mmol) in CH₂Cl₂ (6 ml) was added at 0 °C 3-chloroperoxybenzoic acid (109 mg, 0.633 mmol). The mixture was stirred for 30 min at this temperature and then for 7h at room temperature. This mixture was diluted in CH₂Cl₂ (3 ml), and washed with NaHCO₃ sat. (3 x 3 ml) and NaCl sat. (1 x 3 ml). The organic phase was dried over Na₂SO₄, filtered and concentrated under reduced pressure. The residue was purified by silica gel column chromatography (hexane-AcOEt, 8:1) to give *rac*-(4*R*,5*S*,6*S*,7*R*)-1-benzyloxy-6-*tert*-butyldimethylsilyloxy-7,10-epoxy-7-methyl-5-trimethylsilyloxy-1-azaspiro[3.5]nona-8-en-2-one (8) as a colourless oil (170 mg, 82%).
*R*_{*f*} *=* 0.50 (TLC, hexane-AcOEt, 3:1); yield, 82%; colourless oil; ^{**1**}**H-NMR** (200MHz, CO(CD₃)₂): δ 7.44-7.35 (5H, m, Ph), 5.02 (1H, *part A syst. AB,* J = 11.1 Hz, OCH₂Ph), 4.93 (1 H, *part B syst. AB,* J = 11.1 Hz, OCH₂Ph), 4.34 (1H, br. s, H-5 or H-6), 3.58 (1H, br. s, H-6 or H-5), 3.17 (1H, m, H-3), 2.87 (2H, s, H-10), 2.44 (1H, d, J = 14.1 Hz, H-3), 0.87 (9H, s, C(CH₃)₃), 0.16 (9H, s, Si(CH₃)₃), 0.02 (3H, s, SiCH₃), 0.01 (3H, s, SiCH₃); **IR** (NaCl,CCl₄): ν 3028, 2957, 2891, 2854, 1781, 1494, 1472, 1407, 1362, 1255, 1151, 1100, 1055, 994, 881, 842, 803, 778, 753, 666 cm⁻¹; **LRMS**(API-ES⁺): m/z 1003(2M+Na)⁺, 513(M+Na)⁺, 490(M+H)⁺.

### Example 7

### Preparation of rac-(4R,5S,6S,8S,9S)-1-benzyloxy-6-tert-butyldimethylsilyloxy-9-hydroxy-7-methylen-5-trimethylsilyloxy-1-azaspiro[3.5]nonan-2-on-10,8-carbolactone (9)

### Method A.

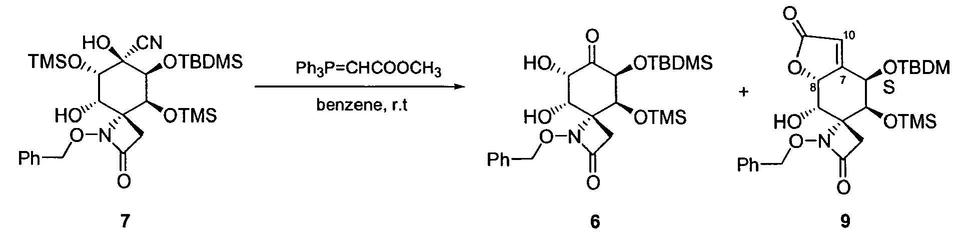

To a solution of *rac*-(4*R*,5*S*,6*S*,7*S*,8*S*,9*S*)-1-benzyloxy-6-*tert*-butyldimethylsilyloxy-7-cyano-7,9-dihydroxy-5,8-bis(trimethylsilyloxy)-1-azaspiro[3.5]nonan-2-one (7) (58 mg, 0.095 mmol) in benzene (1.0 ml) was added at room temperature a solution of methyl (triphenylphosphoranylidene)acetate (76 mg, 0.228 mmol) in benzene (2 ml). The mixture was stirred to reflux for 11 h. The reaction was quenched with Na₂HPO₄ 0.1M buffer (3 ml) and AcOEt (3 ml). The layers were separated and aqueous phase was extracted with AcOEt (3 x 6 ml). The combined extracts were dried over Na₂SO₄, filtered and concentrated under reduced pressure. The residue was purified by silica gel column chromatography (hexane-AcOEt, 4:1) to give *rac*-(4*R*,5*S*,6*S*,8*S*,9*S*)-1-benzyloxy-6-*tert-*butyldimethylsilyloxy-8,9-dihydroxy-5-trimethylsilyloxy-1-azaspiro[3.5]nonan-2,7-dione (6) as a white solid (19 mg, 38 %) and *rac*-(4*R*,5*S*,6*S*,8*S*,9*S*)-1-benzyloxy-6-*tert-*butyldimethylsilyloxy-9-hydroxy-7-methylen-5-trimethylsilyloxy-1-azaspiro[3.5]nonan-2-on-10,8-carbolactone (9) as a white solid (10 mg, 20 %).

### Method B.

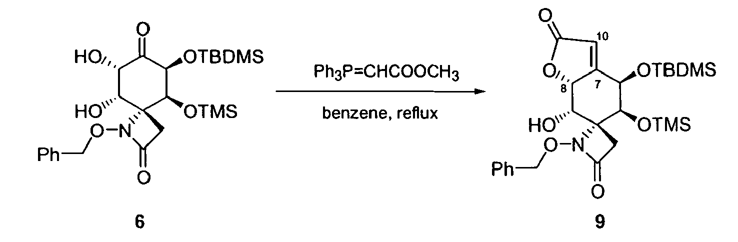

To a solution of *rac*-(4*R*,5*S*,6*S*,8*S*,9*S*)-1-benzyloxy-6-*tert*-butyldimethylsilyloxy-8,9-dihydroxy-5-trimethylsilyloxy-1-azaspiro[3.5]nonan-2,7-dione (6) (59 mg, 0.116 mmol) in benzene (1 ml) was added at room temperature a solution of methyl (triphenylphosphoranylidene)acetate (124 mg, 0.370 mmol) in benzene (3 ml). The mixture was stirred to reflux for 15h. The reaction was quenched with Na₂HPO₄ 0.1M buffer (3 ml) and AcOEt (3 ml). The layers were separated and aqueous phase was extracted with AcOEt (3 x 6 ml). The combined extracts were dried over Na₂SO₄, filtered and concentrated under reduced pressure. The residue was purified by silica gel column chromatography (hexane-AcOEt, 3:1) to give *rac*-(4*R*,5*S*,6*S*,8*S*,9*S*)-1-benzyloxy-6-*tert-*butyldimethylsilyloxy-9-hydroxy-7-methylen-5-trimethylsilyloxy-1-azaspiro[3.5]nonan-2-on-10,8-carbolactone (9) as a white solid (44 mg, 71 %).
*Rf =* 0.14 (TLC, hexane-AcOEt, 3:1), 0.66 (TLC, hexane-AcOEt, 1:1); yield, 71 %; white solid; ^{**1**}**H-NMR** (300MHz, CDCl₃): δ 7.42-7.38 (2H, m, Ph), 7.36-7.33 (3H, m, Ph), 6.00 (1H, d, J = 1.5 Hz, H-10), 5.09 (1H, dd, J = 3.6,1.5 Hz, H-8), 5.08 (1H, *part A syst. AB,* J = 10.2 Hz, OCH₂Ph), 5.03 (1H, *part B syst. AB,* J = 10.2 Hz, OCH₂Ph), 4.77 (1H, d, J = 3.2 Hz, H-5), 4.32-4.27 (2H, m, H-9 and H-6), 3.46 (1H, *part A syst. AB*, J = 13.8 Hz, H-3), 2.37 (1H, d, J = 2.9 Hz, HO-C(9)), 2.32 (1H, *part B syst. AB*, J = 13.8 Hz, H-3), 0.85 (9H, s, C(CH₃)₃), 0.10 (3H, s, SiCH₃), 0.09 (9H, s, Si(CH₃)₃), 0.01 (3H, s, SiCH₃); ^{**13**}**C-NMR** (75MHz, CDCl₃): δ 165.4, 165.1, 135.2, 129.4, 128.8, 128.4, 115.8, 78.8, 78.6, 72.7, 69.5, 67.1, 66.9, 37.6, 25.5, 18.0, 0.4, -4.9 ; **IR** (KBr): ν 3435, 2949, 2927, 2855, 1779, 1747, 1631, 1248, 1129, 1101, 840, 616 cm⁻¹; **LRMS(API-ES**^{**+**}**)**: m/z 1088(2M+Na)⁺, 556(M+Na)⁺, 533(M+H)⁺.

### Example 8

### Preparation of rac-(4R,5S,6S)-1-benzyloxy-6-tert-butyldimethylsilyloxy-7-methylen-5-trimethylsilyloxy-1-azaspiro[3.5]nona-8-en-2-on-10,8-carbolactone (10)

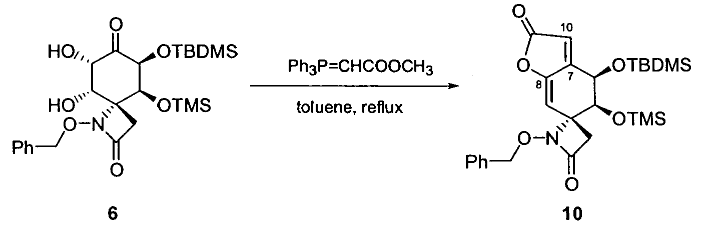

To a solution of *rac*-(4*R*,5*S*,6*S*,8*S*,9*S*)-1-benzyloxy-6-*tert*-butyldimethylsilyloxy-8,9-dihydroxy-5-trimethylsilyloxy-1-azaspiro[3.5]nonan-2,7-dione (6) (19 mg, 0.037 mmol) in toluene (0.3 ml) was added at room temperature a solution of methyl (triphenylphosphoranylidene)acetate (93 mg, 0.277 mmol) in toluene (0.7 ml). The resulting mixture was stirred at reflux for 24 h and then concentrated under reduced pressure. The residue was purified by silica gel column chromatography (hexane-AcOEt, 6:1) to give *rac*-(4*R*,5*S*,6*S*)-1-benzyloxy-6-*tert*-butyldimethylsilyloxy-7-methylen-5-trimethylsilyloxy-1-azaspoo[3.5]nona-8-en-2-on-10,8-carbolactone (10) as a yellow oil (44 mg, 71%).
***Rf** =* 0.55 (TLC, hexane-AcOEt, 3:1); yield, 26%; yellow oil; ^{**1**}**H-NMR** (200MHz, CDCl₃): δ 7.35-7.26 (5H, m, Ph), 5.91 (1H, m, H-9 or H-10), 4.99 (1H, *partA syst. AB,* J = 11.5 Hz, OCH₂Ph), 4.99 (1H, d, J = 1.8 Hz, H-10 or H-9), 4.87 (1H, *part B syst. AB,* J = 11.5 Hz, OCH₂Ph), 4.73 (1 H, d, J = 2.6 Hz, H-5 or H-6), 3.97 (1H, d, J = 2.6 Hz, H-6 or H-5), 3.42 (1H, *part A syst. AB,* J = 13.9 Hz, H-3), 2.47 (1H, *part B syst. AB,* J = 13.9 Hz, H-3), 0.78 (9H, s, C(CH₃)₃), 0.20 (3H, s, SiCH₃), 0.12 (9H, s, Si(CH₃)₃), 0.07 (3H, s, SiCH₃); **LRMS(API-ES**^{**+**}**)**: m/z 538(M+Na)⁺, 516(M+H)⁺.

### Example 9

### Preparation of rac-(4R,5S,6S,7R)-1-benzyloxy-6-tert-butyldimethylsilyloxy-7-hydroxy-7-ethoxycarbonylmethyl-5-trimethylsilyloxy-1-azaspiro[3.5]nona-8-en-2-one (11)

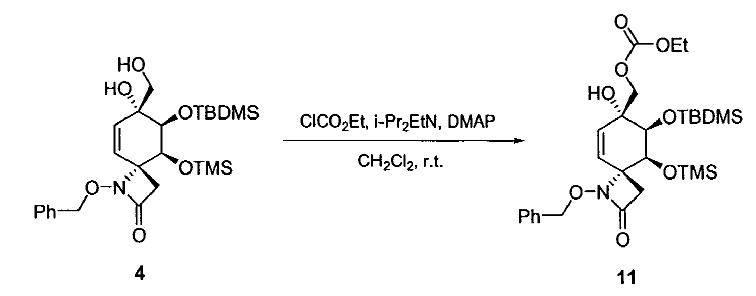

To a solution of *rac*-(4*R*,5*S*,6*S*,7*R*)-1-benzyloxy-6-*tert*-butyldimethylsilyloxy-7-hydroxy-7-hydroxymethyl-5-trimethylsilyloxy-1-azaspiro[3.5]nona-8-en-2-one (4) (52 mg, 0.102 mmol) and DMAP (1.3 mg, 0.010 mmol) in CH₂Cl₂ (1.5 ml) was added at 0 °C N-ethyldiisopropylamine (42 µl, 0.245 mmol) and ethyl chloroformate (12 µl, 0.122 mmol). The resulting mixture was stirred at room temperature until the reaction was complete (16 h, TLC monitoring, hexane-AcOEt, 1:1). The reaction was quenched with H₂O (drops), AcOEt (1.5 ml) and saturated NH₄Cl/NH₄OH solution (1.5 ml). The layers were separated and aqueous phase was extracted with AcOEt (3 x 3 ml). The combined extracts were washed with saturated NH₄Cl/NH₄OH solution (5 ml), dried over Na₂SO₄, filtered and concentrated under reduced pressure to give *rac*-(4*R*,5*S*,6*S*,7*R*)-1-benzyloxy-6-*tert-*butyldimethylsilyloxy-7-hydroxy-7-ethoxycarbonylmethyl-5-trimethylsilyloxy-1-azaspiro[3.5]nona-8-en-2-one (11) as a low melting point pale yellow solid (50 mg, 85%).
***Rf*** = 0.32 (TLC, hexane-AcOEt, 3:1), 0.73 (TLC, hexane-AcOEt, 1:1); yield, 85%; pale yellow solid; ^{**1**}**H-NMR** (200MHz, CDCl₃): δ 7.42-7.29 (5H, m, Ph), 5.48 (1H, d, J = 10.1 Hz, H-8 or H-9), 5.42 (1H, m, H-8 or H-9), 4.98 (1H, *part A syst. AB, J* = 11.3 Hz, OCH₂Ph), 4.85 (1H, *part B syst. AB*, J = 11.3 Hz, OCH₂Ph), 4.55 (1H, br. s, H-5 or H-6 or H-10), 4.22 (1H, q, J = 14.4, 7.1 Hz, OCH₂CH₃), 4.22 (1H, q, J = 14.2, 7.1 Hz, OCH₂CH₃), 4.13 (1H, br. s, H-6 or H-10 or H-5), 3.88 (1H, br. s, H-10 or H-5 or H-6), 3.23 (1H, *part A syst. AB*, J = 13.4 Hz, H-3), 2.27 (1H, br. s, H-10 or H-5 or H-6), 2.26 (1H, *part B syst. AB,* J = 13.4 Hz, H-3), 1.32 (3H, t, J = 7.1 Hz, OCH₂CH₃), 0.80 (9H, s, C(CH₃)₃), 0.18 (9H, s, Si(CH₃)₃), 0.15 (3H, s, SiCH₃), 0.06 (3H, s, SiCH₃); **LRMS(API-ES**^{**+**}**):** m/z 1181(2M+Na)⁺, 602(M+Na)⁺, 580(M+H)⁺.

### Example 10

### Preparation of rac-(4R,5S,6S,7R)-1-benzyloxy-6-tert-butyldimethylsilyloxy-7-methyl-5-trimethylsilyloxy-7,10-phenylboranediyldioxy-1-azaspiro[3.5]nona-8-en-2-one (12)

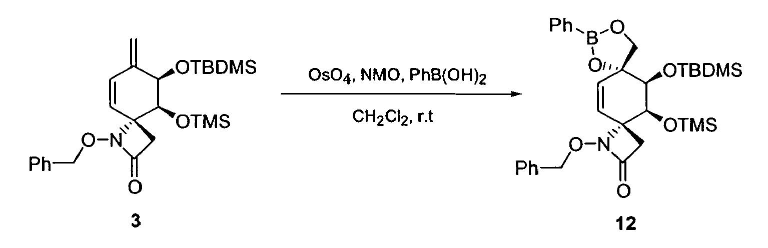

To a solution of *N*-methylmorpholine *N*-oxide (18 mg, 0.149 mmol) and phenylboric acid (19 mg, 0.149 mmol) in CH₂Cl₂ (0.25 ml) was added sequentially at room temperature osmium tetroxide (32 µl, 0.5 mM solution in CH₂Cl₂, 0.0025 mmol) and a solution of *rac-*(4*R*,5*S*,6*S*)-1-benzyloxy-6-*tert*-butyldimethylsilyloxy-7-methylen-5-trimethylsilyloxy-1-azaspiro[3.5]nona-8-en-2-one (3) (59 mg, 0.124 mmol) in CH₂Cl₂ (0.5 ml). The resulting mixture was stirred at room temperature until the reaction was complete (2 h, TLC monitoring, hexane-AcOEt, 3:1), and then quenched with 10% aqueous Na₂S₂O₃ solution (0.5 ml). After 15 min, the mixture was extracted with AcOEt (3 x 2 ml) and the combined organic extracts were dried over Na₂SO₄, filtered and concentrated under reduced pressure. The residue was purified by silica gel column chromatography (hexane-AcOEt, 5:1) to give *rac*-(4*R*,5*S*,6*S*,7*R*)-1-benzyloxy-6-*tert*-butyldimethylsilyloxy-7-methyl-5-trimethylsilyloxy-7,10-phenylboranediyldioxy-1-azaspiro[3.5]nona-8-en-2-one (12) as a low melting point white solid (33 mg, 45%).
***Rf =*** 0.40 (TLC, hexane-AcOEt, 3:1); yield, 45%; white solid; ^{**1**}**H-NMR** (200MHz, CDCl₃): δ 7.84 (2H, dd, J = 8.1, 1.5 Hz, Ph), 7.56-7.30 (8H, m, Ph), 6.91, 5.52 (1H, m), 5.52 (1H, m), 5.07 (1H, *part A syst. AB,* J = 10.1 Hz, OCH₂Ph), 4.93 (1H, *part B syst. AB,* J = 10.1 Hz, OCH₂Ph), 4.71 (1H, br. s), 4.60-4.30 (1H, m), 4.10-3.80 (2H, m), 3.26, 2.33 (1H, br. s), 2.59 (1H, br. s), 0.80 (9H, s, C(CH₃)₃), 0.17 (9H, s, Si(CH₃)₃), 0.10 (3H, s, SiCH₃), 0.07 (3H, s, SiCH₃).

### Example 11

### Preparation of rac-(4R,5S,6S,7R)-1-benzyloxy-6,7-bis(tert-butyldimethylsilyloxy)-7-hydroxy-5-trimethylsilyloxy-1-azaspiro[3.5]nona-8-en-2-one (13)

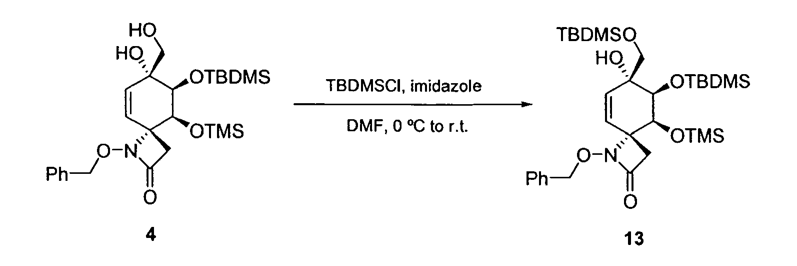

To a solution of *rac*-(4*R*,5*S*,6*S*,7*R*)-1-benzyloxy-6-*tert*-butyldimethylsilyloxy-7-hydroxy-7-hydroxymethyl-5-trimethylsilyloxy-1-azaspiro[3.5]nona-8-en-2-one (4) (50 mg, 0.098 mmol) and imidazole (8 mg, 0.118 mmol) in DMF (0.25 ml) was added at 0 °C a solution of tert-butyldimethylsilyl chloride (18 mg, 0.118 mmol) in DMF (0.25 ml). After 5 h at room temperature, the reaction was quenched with H₂O (1 ml) and the mixture extracted with AcOEt (3 x 2 ml). The combined extracts were washed with saturated aqueous CuSO₄ solution (2 x 3 ml) and brine (2 x 3 ml), dried over Na₂SO₄, filtered and concentrated under reduced pressure. The residue was purified by silica gel column chromatography (hexane-AcOEt, 6:1) to give *rac*-(4*R*,5*S*,6*S*,7*R*)-1-benzyloxy-6,7-bis(*tert*-butyldimethylsilyloxy)-7-hydroxy-5-trimethylsilyloxy-1-azaspiro[3.5]nona-8-en-2-one (13) as a white solid (50 mg, 82%).
***Rf*** = 0.69 (TLC, hexane-AcOEt, 3:1); **yield,** 82%; white solid; ^{**1**}**H-NMR** (200MHz, CDCl₃): δ 7.44-7.27 (5H, m, Ph), 5.55 (2H, m, H-8 and H-9), 4.97 (1H, *part A syst. AB,* J = 11.1 Hz, OCH₂Ph), 4.88 (1H, *part B syst. AB,* J = 11.1 Hz, OCH₂Ph), 4.62 (1H, br. s, H-5 or H-6), 3.81 (1H, br. s, H-6 or H-5), 3.62 (1H, *part A syst. AB,* J = 9.6 Hz, OCH₂CH₃), 3.54 (1H, *part B syst. AB,* J = 9.6 Hz, OCH₂CH₃), 3.23 (1H, *part A syst. AB, J* = 13.6 Hz, H-3), 2.94 (1H, br. s, OH), 2.26 (1H, *part B syst. AB,* J = 13.6 Hz, H-3), 1.32 (3H, t, J = 7.1 Hz, OCH₂CH₃), 0.92 (9H, s, C(CH₃)₃), 0.81 (9H, s, C(CH₃)₃), 0.17 (9H, s, Si(CH₃)₃), 0.14 (3H, s, SiCH₃), 0.09 (6H, s, SiCH₃), 0.06 (3H, s, SiCH₃).

## Claims

1. A compound of formula I: 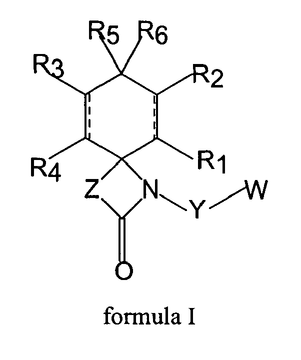 wherein R₁ and R₂ are each independently selected from H, OH or OPR;
R₃ and R₄ are each independently selected from H, OH, OPR, =O, cyano, substituted or unsubstituted alkyl, substituted or unsubstituted aryl, substituted or unsubstituted heterocyclyl, substituted or unsubstituted alkoxy, substituted or unsubstituted aryloxy, substituted or unsubstituted amino or halogen,
R₅ and R₆ together are =O or -O-(CH₂)ₘ-, wherein m is selected from 1, 2, 3, 4 or 5;
or R₅ is selected from H, OH, OPR and R₆ is selected from hydrogen, cyano, substituted or unsubstituted alkyl, substituted or unsubstituted cycloalkyl, substituted or unsubstituted alkenyl, substituted or unsubstituted alkinyl, substituted or unsubstituted aryl, substituted or unsubstituted heterocyclyl,
with the proviso that at least one of R₁, R₂, R₃, R₄ or R₅ is OH or OPR;
PR is an hydroxyl protecting group which can be the same or different on each of R₁, R₂, R₃, R₄ or R₅;
the dotted line represents a single or double bond, with the proviso that when both R₁ and R₂ or R₃ and R₄ are H then there is a double bond between the two C to which the H are linked;
Z is -(CRaRb)ₙ- wherein n is a number selected from 1, 2 or 3 and Ra and Rb are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino or halogen;
Y is selected from -O-, -S-, -N(RaRb)- or -C(O)-, wherein Ra and Rb are as previously defined and do not form a cyclic ring;
W is a group selected from substituted or unsubstituted arylalkyl, substituted or unsubstituted heterocyclylalkyl, substituted or unsubstituted alkenyl;
or a salt, complex or solvate thereof.

2. A compound according to claim 1 **characterized in that** Z is -(CHRa)ₙ-, Ra and n being as defined in claim 1.

3. A compound according to claim 1 or 2 **characterized in that** n is 1.

4. A compound according to any of claims 1-3 **characterized in that** Y is -0-.

5. A compound as defined in anyone of claims 1-4 **characterized in that** W is -CRaRb-Q, wherein Ra and Rb are as previously defined and Q is substituted or unsubstituted aryl, substituted or usubstituted heterocyclyl, substituted or unsubstituted alkenyl.

6. A compound according to claim 1 which corresponds to any of the following formulae: 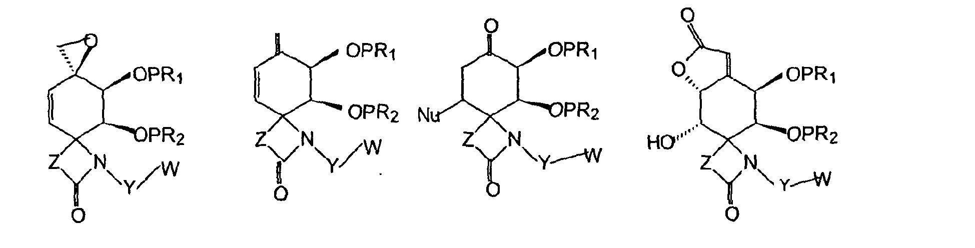 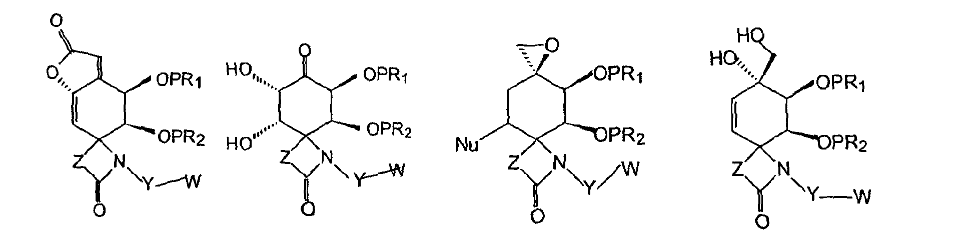 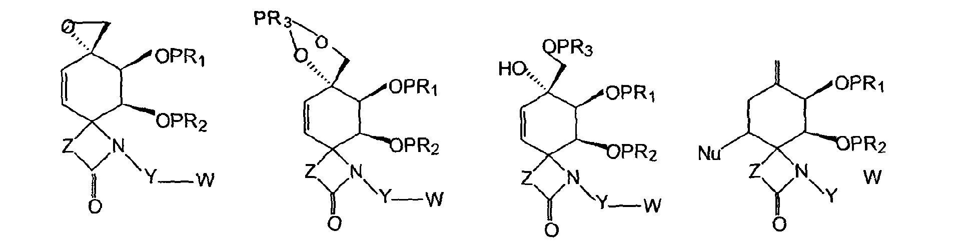 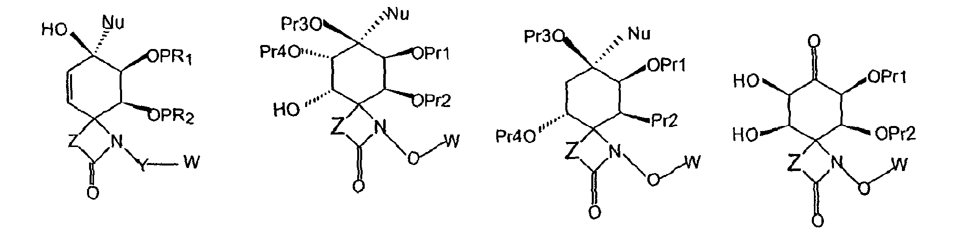 wherein Z, Y and W are as defined in claim 1; PR₁ to PR₅ are hydroxyl protecting groups which can be independently the same or different on each hydroxyl and can optionally protect at the same time two hydroxyl groups, and Nu is a nucleophilic group; their diastereoisomers, enantiomers and mixtures thereof.

7. A process for the preparation of a compound according to any of claims 1-6 which comprises in any order one or more of a step selected from the group consisting of:
a) hydroxylation or dihydroxylation
b) hydroxyl or carbonyl protection
c) nucleophilic attack on the carbonyl group
d) hydroxyl inversion
e) allylic rearrangement
applied to a compound of formula IV: 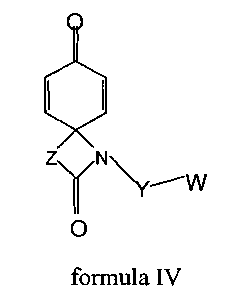 wherein Z , Y and W are as defined in claim 1.
